# EUROPEAN PATENT SPECIFICATION

(11) **EP 1 413 208 B1**
(45) Date of publication and mention of the grant of the patent: **03.01.2007**
(21) Application number: 03009107.8
(22) Date of filing: 19.04.2003
(51) Int. Cl.: A23L 1/30, A61K 36/00

(54) **Use of an antitumor agent**
Verwendung eines Anti-Tumor-Mittels
L'utilisation d'un agent antitumoral

(30) Priority: 24.10.2002 JP 2002309975
(43) Date of publication of application: 28.04.2004
(73) Proprietor: Adaptgen Pharmaceutical Co., Ltd., Ena-gun, Gifu (JP)
(72) Inventor: Hayashi, Hiromichi, Mizunami-city, Gifu (JP)
(74) Representative: Zenz, Joachim Klaus

(56) References cited:
- PATENT ABSTRACTS OF JAPAN vol. 2002, no. 05, 3 May 2002 (2002-05-03) & JP 2002 017248 A (TAKEKKUSU TECHNO:KK), 22 January 2002 (2002-01-22)
- PATENT ABSTRACTS OF JAPAN vol. 2000, no. 21, 3 August 2001 (2001-08-03) & JP 2001 095521 A (KUSAKA HIDEMOTO), 10 April 2001 (2001-04-10)
- DATABASE WPI Section Ch, Week 200029 Derwent Publications Ltd., London, GB; Class D13, AN 2000-333369 XP002251266 & JP 2000 104063 A (KIKUSAKI Y), 11 April 2000 (2000-04-11)
- DATABASE FSTA [Online] INTERNATIONAL FOOD INFORMATION SERVICE (IFIS), FRANFURT/MAIN, DE; MI-JUNG KIM ET AL: "Antibacterial activity of the bamboo (Pseudosasa japonica Makino) leaves extracts on lactic acid bacteria related to dongchimi." Database accession no. 97-1-11-a0065 XP002251264 & JOURNAL OF THE KOREAN SOCIETY OF FOOD SCIENCE AND NUTRITION 1996 CORRESPONDENCE (REPRINT) ADDRESS, MYUNG-SOOK JANG, DEP. OF FOOD SCI. & NUTR., DANKOOK UNIV., SEOUL 140-714, KOREA, vol. 25, no. 5, pages 741-746,
- DATABASE WPI Section Ch, Week 199237 Derwent Publications Ltd., London, GB; Class B04, AN 1992-300577 XP002251267 & CN 1 057 196 A (CHENGDU TRADITIONAL CHINESE MEDICAL COLL), 25 December 1991 (1991-12-25)
- DATABASE MEDLINE [Online] US NATIONAL LIBRARY OF MEDICINE (NLM), BETHESDA, MD, US; January 1998 (1998-01) TSUNODA S ET AL: "Effects of Sasa Health, extract of bamboo grass leaves, on spontaneous mammary tumourigenesis in SHN mice." Database accession no. NLM9568070 XP002251265 & ANTICANCER RESEARCH. GREECE 1998 JAN-FEB, vol. 18, no. 1A, January 1998 (1998-01), pages 153-158, ISSN: 0250-7005
- DATABASE WPI Section Ch, Week 200241 Derwent Publications Ltd., London, GB; Class D15, AN 2002-376086 XP002251268 & JP 2002 035770 A (OTANI M), 5 February 2002 (2002-02-05)
- DATABASE WPI Section Ch, Week 199603 Derwent Publications Ltd., London, GB; Class B04, AN 1996-026971 XP002251269 & JP 07 300424 A (SOGYU KK), 14 November 1995 (1995-11-14)

## Description

### BACKGROUND OF THE INVENTION

### 1. Field of the Invention

The present invention relates to an antitumor agent having an antitumor effect.

### 2. Description of the Related Art

In Japan, it has been customary since ancient times to eat bamboo shoots which grow from bamboo rootstock. Furthermore, powdering fully grown bamboo for use as livestock feed is known.

The abstract of JP-A-2002017248 describes an ethanol-water-extract of *Phyllostachys pubescens.* The extract can be used to retain the freshness of food.

The abstract of JP-A-2001095521 also describes an extract of bamboo which is prepared by cutting the bamboo into pieces, soaking it with an aqueous ethyl alcohol solution containing at least 35% ethanol. The extract can be used as a seasoning.

The abstract of Journal of the Korean Society of Food Science and Nutrition, 1996, vol. 25, no. 5, pg. 741 - 746, describes an extract from bamboo leaves, wherein the extract was obtained by extracting the bamboo leaves in a 70% ethanol solution. According to the abstract, the respective extract has an antibacterial effect.

### SUMMARY OF THE INVENTION

The applicant has discovered that an alcohol extract of bamboo, which may include bamboo sprouts, has an excellent antitumor effect. The present invention is based on this discovery, and an object thereof is to provide an antitumor agent having an antitumor effect.

The aforementioned object is solved by use of an extract extracted from moso bamboo *(Phyllostachys pubescens)* by means of an alcohol solution for the preparation of an antitumor agent. This object is also solved by use of an extract extracted from bamboo by means of an alcohol solution, wherein said bamboo is moso bamboo *(Phyllostachys pubescens)* shoot flesh or the outer part of a fully grown moso bamboo stem for the preparation of an antitumor agent. Alcohol extracts of bamboo have an excellent antitumor effect; accordingly, if a health food comprising such an alcohol extract of bamboo is orally consumed on a regular basis, the progression of malignant tumors (cancers) can be prevented or limited. The antitumor effect of such an alcohol extract is more pronounced with an aqueous alcohol solution, which contains water, than with a 100% alcohol solution. The alcohol concentration of such an aqueous alcohol solution is preferably 50% to 70%, and it is most preferable that the alcohol concentration of this aqueous alcohol solution be 60%. In terms of the bamboo, the flesh of moso bamboo *(Phyllostachys pubescens)* sprouts and the outer part of the stem of this same type of bamboo are particularly preferred.

Furthermore, antitumor agents having the aforementioned extract as an active component thereof are highly effective in the treatment of malignant tumors.

### BRIEF DESCRIPTION OF THE DRAWINGS

FIG. 1 is a graph showing the results (right leg) for trial 1;
FIG. 2 is a graph showing the results (left leg) for trial 1;
FIG. 3 is a graph showing the results for trial 2;
FIG. 4 is a graph showing the results for trial 2;
FIG. 5 is a graph showing the results for trial 2;
FIG. 6 is a graph showing the results for trial 3;
FIG. 7 is a graph showing the results for trial 3;
FIG. 8 is a graph showing the results for trial 3; and
FIG. 9 is a graph showing the results for trial 4.

### DESCRIPTION OF THE PREFERRED EMBODIMENTS

In the following, modes of embodiment of the present invention are described. Firstly, the bamboo which serves as the primary material is moso bamboo, Both fully grown bamboo and bamboo shoots may be used. The parts of the bamboo plant which may be used include the stem, the roots, and the leaves. It is preferable that the bamboo be fresh and that parts of the plant other than the shoot be used within one year of harvest. If bamboo shoots are used, it is preferable that these be processed soon after harvest. In either case, however, if the bamboo is frozen while still fresh, it can be stored for usage at a later date.

Next, the bamboo is washed to remove any impurities, and then ground in a mill to a particle size which passes through a 10 mm screen. For mills that are not fitted with screens, the bamboo may be milled to a particle length of 3 mm to 5 mm. Next, 1 kg of ground bamboo is immersed in 10 kg of an alcohol solution or an aqueous alcohol solution until it is thoroughly bleached. The period of time required for this is normally approximately 6 months. However, if this is heated to approximately 60°C, approximately 2 hours is sufficient.

Next, the solid component is separated in a centrifuge, and the liquid alcohol extract of bamboo is recovered. This liquid extract is concentrated to 20% of the original weight thereof by heating it to 60°C. If an equivalent solid amount of starch is added to this concentrated bamboo extract, this can be freeze-dried to form a powder, which serves as the final health food or antitumor agent. It is a matter of course that vitamins and the like can be added to this powder. The health-food product or antitumor agent may take the form of a solid, a powder, a liquid, granules, or the like.

In the process described above, the dross that remains after centrifuging may be recovered, 6 parts by weight of an alcohol solution or an aqueous alcohol solution may be added to each 1 part by weight of dross, and the liquid produced by heating this to 60°C for 2 hours may be mixed into the liquid produced in the process described above. In this manner, the bamboo can be fully exploited, without waste.

If the antitumor agent described above is to be used as an injectable agent, a resin purification process is further performed, before concentration to 20% of the original volume described in the process above, and the concentrated extract is further deproteinized with ethanol or the like. This is then subject to microfiltration so as to purify it and remove bacteria, whereafter it is autoclaved, for example, at 120°C for 30 minutes. In the case of ethanol deproteinization, the product is concentrated by heating at 80°C to completely remove the alcohol.

As is made clear from the foregoing description, the present invention includes the technical idea of "a method of preparing a health-food product or an antitumor agent comprising a step wherein bamboo is immersed in an alcohol solution or an aqueous alcohol solution, and a step wherein a liquid alcohol extract of this bamboo is recovered and concentrated."

Samples of a product according to the present invention prepared by means of the method described above were tested for antitumor effect in the following trials 1 to 4.

### Trial 1

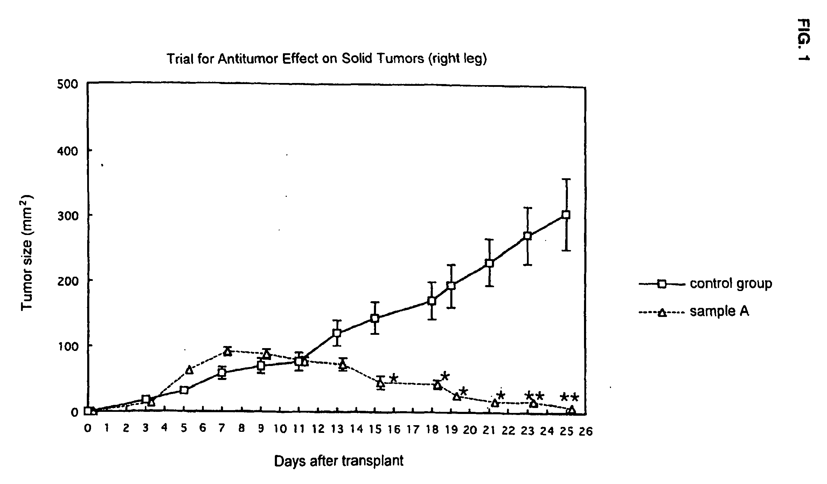

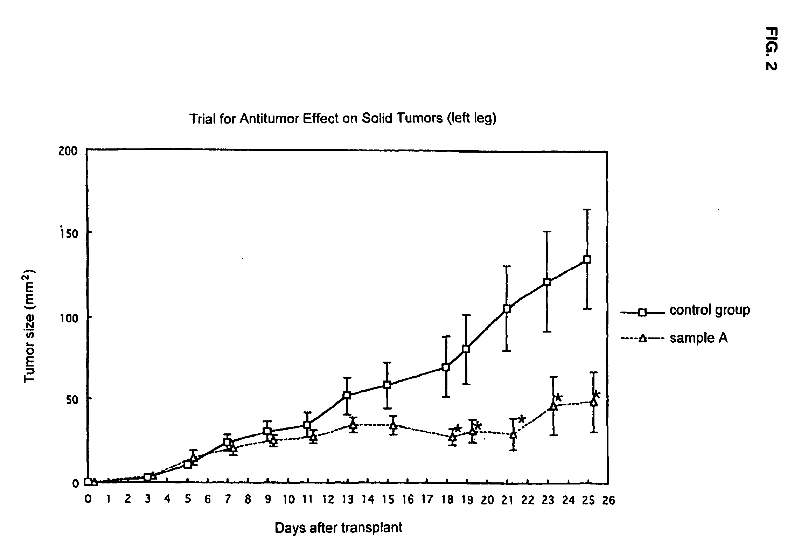

Malignant sarcoma cells (Meth-A fibrosarcoma) were transferred to both subcutaneous inguinal regions of BALB/c mice, a solid tumor was grown, and changes in the surface area of the tumor over time were measured for 25 days. For 3 days, beginning on the third day following the transfer, 0.1 ml of a sample A (a liquid wherein a powder extracted from moso bamboo, using an aqueous alcohol solution having an alcohol concentration of 60%, is dissolved in a physiological saline solution) was administered to the interior of the tumor on the right leg of each mouse. A physiological saline solution was administered under the same conditions to a control group. The content of powder in 0.1 ml of sample A was 1 mg. The results are shown in the graphs in FIG. 1 and FIG. 2.

As is made clear by the trial results shown in FIG. 1 and FIG. 2, tumor growth was suppressed by the administration of sample A in both legs. Changes in the surface area of the tumors in the right leg represent the direct effect of the sample A, while changes in the surface area of the tumors in the left leg are thought to be the result of sensitization and activation of immune functions within the organism as a result of the sample A.

### Trial 2

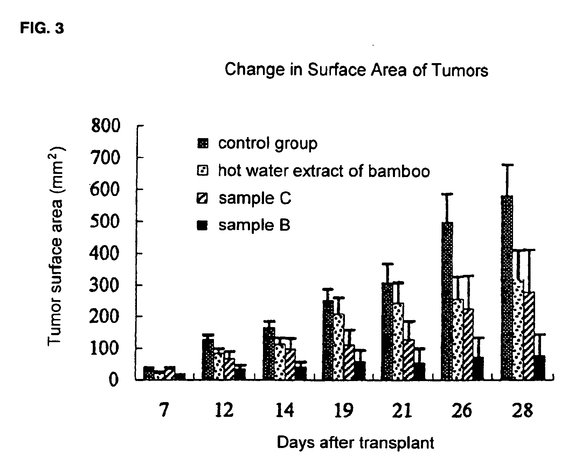

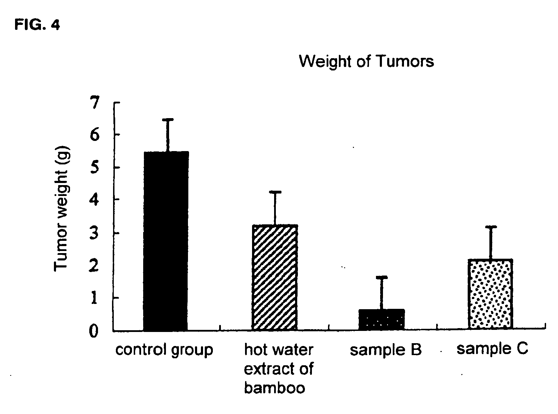

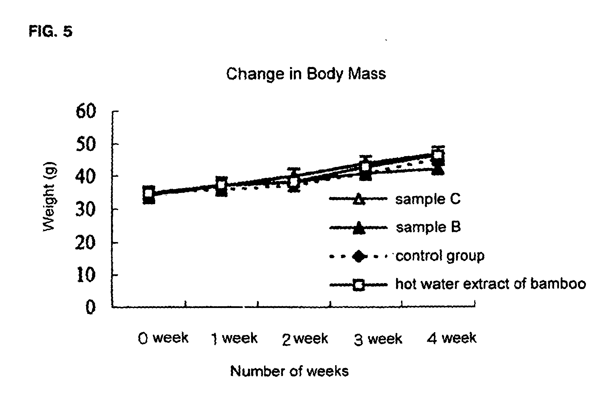

Ascites tumor cells (sarcoma-180) in the amount of 1 x 10⁶ were transferred and grown in the right subcutaneous inguinal region of ICR mice to form solid tumors. A sample B, wherein a powder extracted from moso bamboo with an aqueous ethanol solution having an ethanol concentration of 60% is dissolved in a physiological saline solution, and a sample C, wherein a powder extracted from moso bamboo with a 100% ethanol solution is dissolved in a physiological saline solution, were administered orally at 1000 mg/kg/day beginning 24 hours after transplant. Administration was performed every other day, and the mice were raised for 28 days. Under the same conditions, a physiological saline solution was administered to a control group, and a hot water extract of bamboo was used as a comparative example. Meanwhile, the surface area of the tumors and the changes in body mass of the ICR mice were measured and, on the 28th day, the tumors were excised and weighed. Changes in the surface areas of the tumors are shown in the graph in FIG. 3, changes in the weight of the tumors are shown in the graph in FIG. 4, and changes in body mass are shown in the graph in FIG. 5.

As is made clear from the results shown in FIG. 3, the values for both sample B and sample C are significantly lower than for the control group from day 12 to day 28. As compared to this, the values for the hot water extract of bamboo were only significantly lower than those of the control group on day 26, but a significant difference was not found on day 28. Furthermore, as shown in the graph in FIG. 4, the values for the weights of the excised tumors were significantly lower for samples B and C than for the control group, but a significant difference was not found between the control group and the hot water extract of bamboo. These trial results made it clear that samples of B and C inhibited tumor growth without impairing body growth, demonstrating an excellent antitumor effect. As is made clear from the graph in FIG. 5, there were no significant differences in body weight between any of the groups during the trial period, and neither sample affected the growth of the mice.

### Trial 3

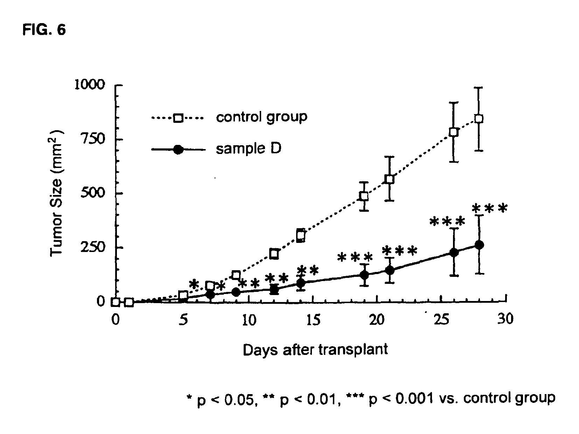

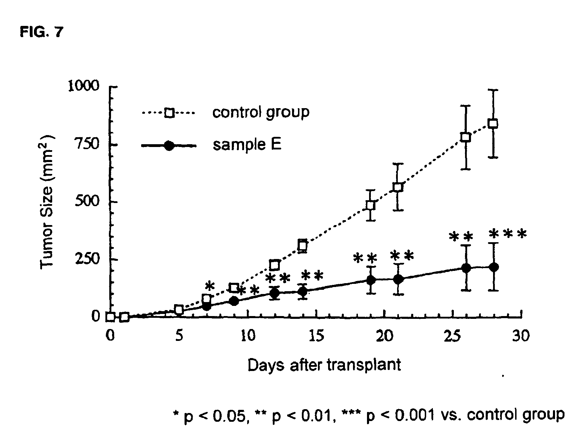

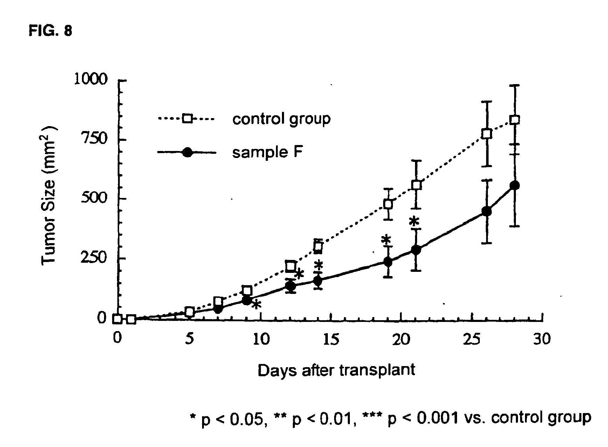

Ascites tumor cells (sarcoma-180) in the amount of 1.2 x 10⁶ were transferred to the right subcutaneous inguinal region of ICR mice and grown to form solid tumors. Beginning 24 hours after the transplant, a sample D, wherein a powder extracted from bamboo shoots using a 60% aqueous ethanol solution was dissolved in a physiological saline solution, and a sample E, which was produced in like manner from bulk bamboo (a mixture of approximately equal amounts of Japanese timber bamboo, moso bamboo, black bamboo *(Phyllostachys nigra var*. *nigera*), arrow bamboo *(Pseudosasa japonica)* hachiku bamboo, medake bamboo *(Pleioblastus simonii)*, all of which are grown in Japan, and Indonesian Tali bamboo), and a sample F, produced in like manner from Indonesian Tali bamboo, were administered orally at 1000 mg/kg/day. Administration was performed every other day, and for each group, nine mice were raised for 28 days. A physiological saline solution was administered under the same conditions to a control group. During this time, the surface areas of the tumors were measured over time. The tumors were excised and weighed on the 28th day. The results are shown in Table 1 and in the graphs in FIG. 6 to FIG. 8. In Table 1, the tumor inhibition rate (%) is found by the formula [1-(weight of tumors in the group to which the product according to the present invention was administered/weight of the tumors in the control group)] x100, based on the weight of the tumors on the last day.

**Table 1**

| **Trial of Antitumor Effect on Ascites Tumor Cells** | | | |
|---|---|---|---|
| | Average Tumor Weight (g/SE) | Tumor Inhibition Rate (%) | Number of Cases Cured |
| Sample D (shoots) | 1.79 ±0.78* | 78.2 | 3/9 |
| Sample E (bulk) | 1.76 ±0.87* | 78.5 | 4/9 |
| Sample F (Tali) | 5.24 ±1.71 | 36.1 | 219 |
| Control (saline) | 8.20 ±1.82 | | 0/9 |

| | | | |
|---|---|---|---|
| * p < 0.01 compared to control group | | | |

From this trial 3 it was understood that while the strength differed according to the type of bamboo, all of samples D to F had excellent antitumor effects as compared with the control groups.

### Trial 4

Moso bamboo (sample G), the outer part of the fully grown stem of moso bamboo (sample H), the inner part of the fully grown stem of moso bamboo (sample I) (note: the outer part of the stem is a thin portion representing approximately one-half of the stem, and the inner part is a thick portion representing approximately one-half of the stem), the flesh of the bamboo shoot, which is the inner part of the shoot with the skin removed and is the part commonly used for cooking (sample J), and bamboo shoot skin (sample K) were immersed in aqueous ethanol solutions with 60% ethanol concentrations, which were heated to 60°C for two hours, whereupon the liquid extract was filtered, vacuum concentrated (60°C), and dried (60°C). Note that for each 1 part of sample H, 10 parts of ethanol solution were used, and for each 1 part of the other samples, 5 parts of ethanol solution was used.

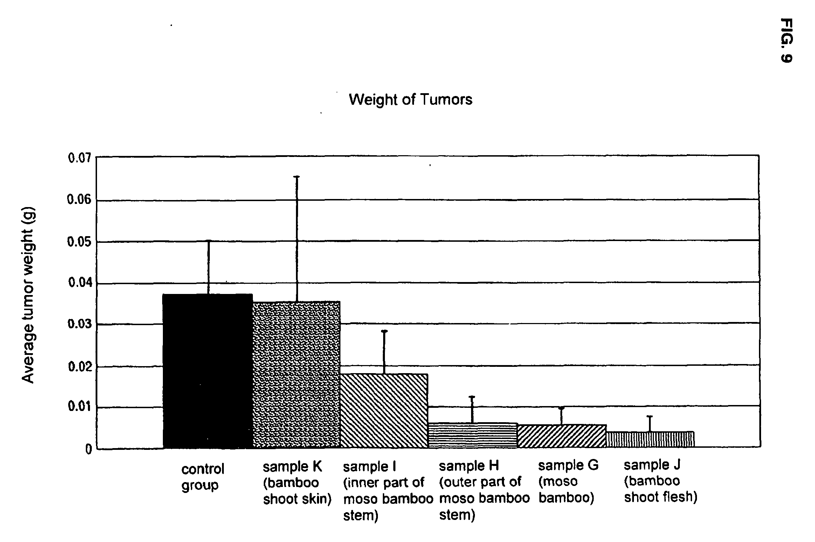

Sarcoma-180 cells in the amount of 1 x 10⁷ cells/ml were transplanted into the right lower abdomen of ICR mice (males, five weeks old), and on day 0 after the transplant, normal feed (Nosan Corporation, Labo MR Breeder^{™}) was given to the control group, while the test group was allowed to feed freely from feed to which the samples G to K had been admixed at a ratio of 20%. Each group contained 10 animals. On the 28th day after transplant, the tumors were excised and weighed. The mean weights of the tumors are shown in FIG. 9.

From this trial 4 it was understood that the flesh of bamboo shoots (sample J) was much more effective than the skin of bamboo shoots (sample K). Furthermore, for the same type of bamboo, the outer part of the fully grown stem (sample H) was more effective than the inner part of the fully grown stem (sample I). Moso bamboo was found to be highly effective.

Ethanol was used for the alcohol solution, but methanol and other alcohol solutions may be used. Furthermore, in this mode of embodiment, an aqueous alcohol solution having an alcohol concentration of 60% was used, but for mass production, an aqueous alcohol solution having an alcohol concentration of 50% to 70% may be used.

## Claims

1. Use of an extract extracted from moso bamboo *(Phyllostachys pubescens)* by means of an alcohol solution for the preparation of an antitumor agent.

2. Use of an extract extracted from bamboo by means of an alcohol solution, wherein said bamboo is-moso bamboo *(Phyllostachys pubescens)* shoot flesh or the outer part of a fully grown moso bamboo stem, for the preparation of an antitumor agent.

3. Use of an extract according to claim 1 or 2 wherein the extract is extracted from bamboo by means of an aqueous alcohol solution having an alcohol concentration of 50% to 70%.

4. Use of an extract according to claim 1 or 2 wherein the extract is extracted from bamboo by means of an aqueous alcohol solution having an alcohol concentration of 60%.

## Patentansprüche

1. Verwendung eines Extraktes, das aus Moso-Bambus *(Phyllostachys pubescens)* mit einer Alkohollösung extrahiert wurde, für die Herstellung eines Antitumormittels.

2. Verwendung eines Extraktes, das aus Bambus mit einer Alkohollösung extrahiert wurde, wobei der Bambus Sprossenfleisch oder der äußere Teil eines vollständig ausgewachsenen Moso-Bambusstammes von Moso-Bambus *(Phyllostachys pubescens)* ist, für die Herstellung eines Antitumormittels.

3. Die Verwendung eines Extraktes gemäß Anspruch 1 oder 2, wobei das Extrakt mit einer wässrigen Alkohollösung mit einer Alkoholkonzentration von 50 bis 70% extrahiert wird.

4. Die Verwendung eines Extraktes gemäß einem der Ansprüche 1 oder 2, wobei der Extrakt mit einer wässrigen Alkohollösung mit einer Alkoholkonzentration von 60% aus Bambus extrahiert wird.

## Revendications

1. Utilisation d'un extrait, extrait de bambou géant *(Phyllostachys pubescens)* au moyen d'une solution alcoolique, pour la préparation d'un agent antitumoral.

2. Utilisation d'un extrait, extrait de bambou au moyen d'une solution alcoolique, dans laquelle ledit bambou est la chair de pousse de bambou géant *(Phyllostachys pubescens)* ou la partie externe d'une tige de bambou géant entièrement développée, pour la préparation d'un agent antitumoral.

3. Utilisation d'un extrait selon la revendication 1 ou 2, dans laquelle l'extrait est extrait de bambou au moyen d'une solution alcoolique aqueuse ayant une concentration en alcool de 50 % à 70 %.

4. Utilisation d'un extrait selon la revendication 1 ou 2, dans laquelle l'extrait est extrait de bambou au moyen d'une solution alcoolique aqueuse ayant une concentration en alcool de 60 %.
